# EUROPEAN PATENT APPLICATION

(11) **EP 2 596 748 A1**
(43) Date of publication of application: **29.05.2013**
(21) Application number: 11190054.4
(22) Date of filing: 22.11.2011
(51) Int. Cl.: A61B 5/07, A61B 1/04, A61B 5/145

(54) **Capsule device for in vivo imaging and pH measuring of the esophagus**

(71) Applicant: King Saud University, 11421 Riyadh (SA)
(72) Inventor: Al Malki, Khalid Hassan Husain, 11421 Riyadh (SA)
(74) Representative: Goddar, Heinz J.

(57) **Abstract**

The present invention relates to a capsule device *for in vivo* imaging and pH measuring of the esophagus, said capsule device having an optical window (1) and comprising at least one illumination source (2) for illuminating an esophagus site; at least one imaging camera (3); an optical system (4) for imaging the esophagus site onto the imaging camera (3); a transmitter (5) for transmitting pH measurement and video output of the imaging camera (3); a pH electrode (8) and a reference electrode (9); means for transmitting pH measuring data to a recording and/or analyzing unit; and at least one fixing means (10) for fixing the capsule device preferably at the mucosa of the esophagus. Optionally, the capsule device can comprise or can be connected to a pressure transducer catheter.

## Description

The present invention relates to a capsule device for *in vivo* imaging and pH measuring of the esophagus.

Gastroesophageal reflux disease (GERD) as a common disorder that affects up to 20% of the population worldwide. In this disease, gastric contents (mainly acids) reflux up into the esophagus or may reach the pharynx and larynx (Laryngopharyngeal reflux disease).

Transient lower esophageal sphincter relaxation (TLESR) has been identified as an important factor in the pathophysiology of GERD. In spite of this, little is known about its details. TLESR is characterized by rapid lower esophageal sphincter (LES) relaxation (opening) in the absence of swallowing or esophageal peristalsis.

The publication of Pandolfino et al. Gastroenterology, 2006, 131, 1725-1733, relates to the mechanistic analysis of transient lower esophageal sphincter relaxations and reflux using concurent fluoroscopy and high-resolution manometry. In detail, the mechanics were investigated leading to esophagastric junction opening during transition lower esophageal sphincter relaxations.

WO 2005/112460 discloses a system and a method for obtaining a sample from, for example, endo-luminal areas. One or more devices may include an image and a transmitter for transmitting in vivo data, and a sampling mechanism for, e.g., example obtaining biopsies.

The publication of Moglia et al., Recent Patents on Biomedical Engineering, 2008, 1, 24-33, reviews the most recent and significant inventions under wireless capsule endoscopy.

US 7,374,547 relates to a wireless esophageal pH-monitoring system which is commercially available as BRAVO® pH. This measurement system is attached on the luminal surface of the lower end of the esophagus by endoscopy. The achieved data are transmitted to a small receiver which the patient is carrying.

PillCam™ ESO 2 Video Capsule is a commercial available endoscopic imaging device which contains an imaging device and a light source. Images are taken while the capsule passes down the esophagus. Images are transmitted from the PillCam to a recording device. It represents an alternative to traditional endoscopy, the most common procedure for examining the esophagus.

It is an object of the present invention to provide a device for studying the causes of gastroesophageal reflux disease which overcomes the drawbacks of the prior art. Particularly, a device shall be provided which allows exhaustive examination of the correlation between esophageal pH, TLESR and GERD.

This object is achieved by a capsule device for *in vivo* imaging and pH measuring of the esophagus, said capsule device having an optical window and comprising at least one illumination source for illuminating a esophagus site; at least one imaging camera; an optical system for imaging the esophagus site onto the imaging camera; a transmitter for transmitting video output of the imaging camera; and a pH electrode, a reference electrode; means for transmitting pH measuring data to a recording and/or analyzing unit; and at least one fixing means for fixing the capsule device at the mucosa of the esophagus.

In a preferred embodiment, the imaging camera is a complementary metal oxide semiconductor (CMOS) type or a charged-coupled device (CCD) type.

Preferred, the illumination source is at least one light-emitting diode, preferably a white light-emitting diode.

Even preferred, the optical system comprises at least one lens, more preferably, a short focal lens, and optionally mirrors and/or prisms.

In a preferred embodiment, the emitter is connected with an antenna.

In a preferred embodiment, the fixing means comprises an evacuable well and a pin.

Preferably, the capsule device of the present invention also comprises a power source, more preferably at least one battery.

Even preferred, the pH electrode is positioned at the capsule device to allow direct contact with environment surrounding the capsule device.

Preferably, the pH electrode is an antimony pH electrode.

In a preferred embodiment, the inventive capsule device is further comprising a pressure transducer catheter.

Preferably, imaging components and pH measuring components are integrated within one capsule or imaging components and pH measuring components are separated into two capsules connected to each other.

Even preferred, the dimension of each capsule is below 1 cm in width and height and below 3.5 cm in length, preferably below 0.7 cm in width and height and 3.0 cm in length, most preferred about 0.5 cm in width and height and about 2,5 cm in length.

Surprisingly, it was found by the inventors that the capsule device of the present invention enables detailed studies of the influence of TLESR on GERD in correlation with the esophageal pH.

It was further surprisingly found that the device of the present invention allows long-term investigations of a specific part of the esophagus.

The device of the present invention can advantageously be used together with a device delivery system (similar to that in the BRAVO® pH system), an external recording device and a work station running an appropriate software for monitoring the generated data.

The inventive device is a single use device. The captured images are transmitted immediately to an external recording device worn by the patient. This recording device is assigned to be compact, easily to use and handle and to remain with the patient as part of an ambulatory examination for 24-48 hours. The images received and stored by the recording device are transferred after the examination to the work station with a software that supports the recorder, transfers its data to local memory, compiles data into a video and reviews or reports functions for the physician.

The imaging camera should be capable to take at least 24 images per second.

The batteries of the esophageal device should be capable of providing power to the system to function for 24-48 hours.

For example, as an external recording device a jacket having communication function as disclosed in US 7,109,933 can be used.

The inventive device is placed in the esophagus transorally and is carried to the right position. A particular advantageous position for placing the inventive device to study the influences of TLESR on GERD is by example about 5 cm above the lower esophageal sphincter. Nevertheless, the device can also be fixed at the mural surface of the upper part of the esophagus with its camera pointing to the upper esophageal sphincter. This could help in understanding the role of the upper esophageal sphincter in GERD.

When fixed above the lower esophageal sphincter another camera can be added at the proximal side of the device to view the swallowed bolus before passing the esophagogastric junction.

Also, the capsule device according to the present invention can be applied to the patient simultaneously with other investigation tools. The inventive capsule device may passively pass through the entire GI tract when detached from the fixing position.

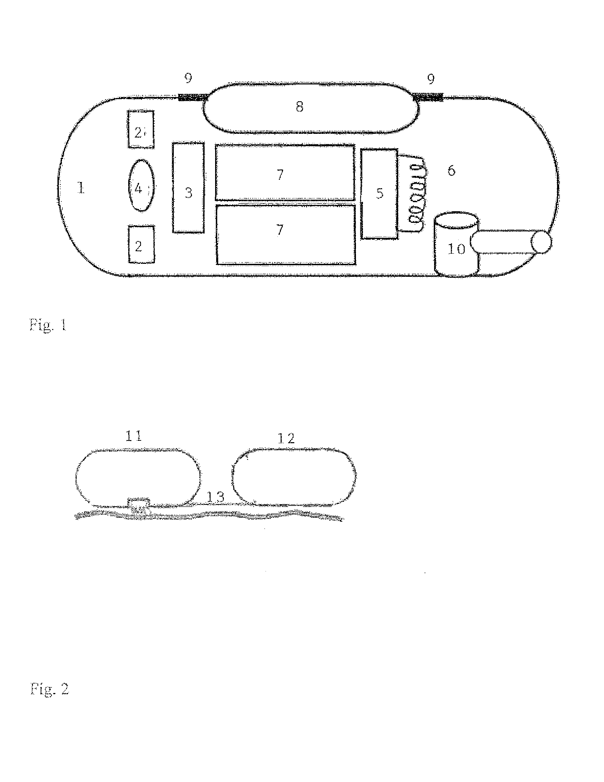

Further characteristics and advantages of the invention result from the detailed description of the preferred embodiments particular in context with the attached drawings, wherein
Fig. 1 is a schematic longitudinal cross section illustration of the capsule device according to one embodiment of the present invention.
Fig, 2 is a schematic illustration of the system in accordance with an embodiment of the present invention wherein the imaging components and pH measuring components are separated into two capsules connected to each other.

### Detailed description of the invention

The capsule device of the present invention is utilized for in vivo imaging and pH measuring of the esophagus and for transmitting the generated data.

Reference is now made to Fig. 1 which illustrates the capsule device and its components, according to an embodiment of the invention. The capsule device typically comprises an optical window 1 and an imaging system for obtaining images from inside of the esophagus. The imaging system includes an illumination source 2, such as a white LED, an imaging camera 3, which detects the images and an optical system 4 which focuses the images onto the imaging camera 3. The illumination source 2 illuminates the inner portions of the esophagus through the optical window 1. The capsule device further includes a transmitter 5 and an antenna 6 for transmitting the video signal of the image camera 3, and a power source 7, such as a battery, that provides power to the electrical elements of the capsule device.

The capsule device comprises further a pH electrode 8 which is positioned at the capsule device to allow direct contact with the environment surrounding the capsule device. The pH electrode 8 is further in electrical contact with a reference electrode 9. Additionally, the capsule device comprises a means which can be identically with the transmitter 5 for transmitting the pH measurement data to a recording and/or analyzing unit.

Finally, the capsule device contains a fixing means 10 which preferably comprises an evacuable well and a pin. The capsule device can be placed in the esophagus transorally in a way similar to the BRAVO® system. After placement of the probe in the right position a continuous vacuum allows to suck a small piece of the mucosa into the well. After that the pin is drawn through the whole and in this way also through the esophageal mucosa.

It will be appreciated that a plurality of imaging cameras may be used in the capsule device of the present invention. Each imaging camera may include its own optical system and either one or more illumination sources, in accordance with specific requirements of the capsule device.

Images obtained by the imaging camera 3 are transmitted to a receiving system (not shown), which may also include a data processing unit. The receiving system and data processing unit are typically located outside a patient.

Besides this, the device may be introduced into an esophagus by using an endoscopic device.

Illumination source 2 transmits illumination to the walls of the esophagus via the optical window 1. The lens of the optical system 4 then focuses remitted light onto the pixels of the imaging camera 3.

The optical system 4 comprises at least one lens and optionally mirrors and/or prisms for collecting and collimating remitted light onto the pixels of the imaging camera 3. Typically, the optical system comprises an aspheric focusing lens.

A single or plurality of light sources or a specific integrated light source may be used and positioned in accordance with specific imaging requirements, such as to avoid stray light etc., Also, the optical window 1 may be positioned and shaped according to the device shape and according to specific imaging requirements. For example, optimized imaging conditions can be obtained when optical window 21 is formed to define an ellipse-shaped dome and the imaging camera 3 and illumination sources 2 are positioned in the proximity of the focal plain of the shape defined by the optical dome.

A suitable transmitter may comprise a moderator which receives the video signal (either digital or analog) from the imaging camera 3, a radio frequency amplifier and an impedance matcher.

Reference is now made to Fig. 2 which schematically illustrates a plurality of connected capsules 11 and 12 in accordance with an embodiment of the invention. The plurality of capsules may be connected by, for example, a thread, tube, cable, wire or flexible narrow shaft 13. According to some embodiments more than one connecting wire or shaft may be used to connect two or more capsules. The connecting wire 13 may physically and/or electrically connect the two or more capsules and may be of any suitable lengths from a few millimeters to a centimeter or more. The flexible connection between the two capsules may make the capsules more flyable and maneuverable in an esophagus than would be a single rigid or partially flexible capsule device of the same size or mass. In this embodiment, the first (proximal or upper) capsule 11. may contain the components necessary for pH measurement and the component for fixing the capsule device to the mural surface of the esophagus, wherein the second (distal or lower) capsule 12 may contain the components necessary for imaging of the esophagus.

The features disclosed in the foregoing description, the claims and the accompanying drawings may, both separately and in any combinations thereof, be material for realizing the invention in diverse forms thereof.

## Claims

1. Capsule device for *in vivo* imaging and pH measuring of the esophagus, said capsule device having an optical window (1) and comprising
at least one illumination source (2) for illuminating an esophagus site;
at least one imaging camera (3);
an optical system (4) for imaging the esophagus site onto the imaging camera (3);
a transmitter (5) for transmitting video output of the imaging camera (3);
a pH electrode (8) and a reference electrode (9);
means for transmitting pH measuring data to a recording and/or analyzing unit; and
at least one fixing means (10) for fixing the capsule device at the mucosa of the esophagus.

2. Capsule device according to claim 1, wherein the imaging camera (3) is a complementary mental oxide semiconductor (CMOS) type or a charged-coupled device (CCD) type.

3. Capsule device according to claim 1 or 2, wherein the illumination source is at least one light-emitting diode (2), preferably a white light-emitting diode.

4. Capsule device according to any of the preceding claims, wherein the optical system comprises at least one lens (4), preferably a short focal lens, and optionally mirrors and/or prisms.

5. Capsule device according to any of the preceding claims, wherein the transmitter (5) is connected with an antenna (6).

6. Capsule device according to any of the preceding claims, wherein the fixing means (10) comprises an evacuable well and a pin.

7. Capsule device according to any of the preceding claims, further comprising a power source (7), preferably at least one battery.

8. Capsule device according to any of the preceding claims, wherein the pH electrode (8) is positioned at the capsule device to allow direct contact with environment surrounding the capsule device.

9. Capsule device according to any of the preceding claims, wherein the pH electrode (8) is an antimony pH electrode.

10. Capsule device according to any of the preceding claims, further comprising or being connected to a pressure transducer catheter.

11. Capsule device according to any of the preceding claims, wherein imaging components and pH measuring components are integrated within one capsule, or imaging components and pH measuring components are separated into two capsules connected to each other.

12. Capsule device according to any of the preceding claims, wherein the dimension of each capsule is below 1 cm in width and height and below 3.5 cm in length, preferably below 0.7 cm in width and height and 3.0 cm in length, most preferred about 0,5 cm in width and height and about 2.5 cm in length.
